# EUROPEAN PATENT APPLICATION

(11) **EP 4 750 101 A1**
(43) Date of publication of application: **27.05.2026**
(21) Application number: 24214476.4
(22) Date of filing: 21.11.2024
(51) Int. Cl.: H04W 4/50, H04W 4/70, H04W 48/20, A61B 5/08, A61M 15/00, H04L 41/0806, H04L 41/0853, H04W 84/12

(54) **MEDICAL DEVICE WITH REMOTE CONNECTIVITY SETUP**

(71) Applicant: Pari Medical Holding GmbH, 82319 Starnberg (DE)
(72) Inventor: Kellerer, Christoph, 82256 Fürstenfeldbruck (DE); Han, Gel, 80339 München (DE); Fuchs, Carola, 82061 Neuried (DE); Finke, Matthias, 82152 Planegg (DE)
(74) Representative: Hoffmann Eitle

(57) **Abstract**

Provided is a medical device, comprising: at least one sensor for generating data related to a usage and/or operation of the medical device and/or data of one or more measured parameters of a user; a connectivity controller configured to: receive access data to a wireless access point from the communication device, and establish a wireless communication connection with the wireless access point using the received access data to wireless transmit the generated data.

## Description

### Technical Field

The present disclosure relates to a medical device and a communication device. In particular, the present disclosure relates to a medical device and a communication device implementing a remote connectivity setup mechanism.

### Background

A medical device is typically a compact, lightweight, and often battery-operated piece of medical equipment for use in various settings inside or outside traditional healthcare facilities. A medical device may, for example, be a portable and/or handheld and/or transportable medical device. These medical devices may enable continuous monitoring, diagnostics, and/or treatment for patients in home care, emergency situations, or remote locations. Examples include portable oxygen concentrators, blood glucose meters, heart rate monitors, handheld ultrasound devices, and wearable electrocardiograms (ECGs), inhalators, nebulizers and the like. By providing real-time data and facilitating timely medical interventions, portable medical devices may significantly enhance patient mobility, improve access to healthcare, and support personalized medical care, ultimately contributing to better health management and outcomes.

A medical device may be connected via a wireless interface, for example a Bluetooth interface, to other digital computing/communication devices. Connecting a medical device may be used to synchronize the medical device with another (mobile) communication device, such as a portable computer, a notebook, a desktop, a smartphone, a tablet, a wearable device or other digital platforms such as cloud services or servers allowing for real-time monitoring, data exchange and management of medication usage. Using an inhalation device as an example, patients can receive reminders and track their inhalation device usage patterns through, for example, a dedicated mobile application (also referred to as APP) running at the communication device, while healthcare providers may gain access to detailed data on adherence and inhalation techniques. The inhalation device may also transmit data to electronic health records (EHRs) for comprehensive patient management. By leveraging wireless connectivity, the inhalation device may not only ensure that patients with asthma, cystic fibrosis or chronic obstructive pulmonary disease (COPD) or the like adhere to their prescribed treatment plans but also provide guidance that can help tailor individual care plans, enhance patient engagement, and improve overall respiratory health outcomes.

### Summary

### Technical Problem

A medical device, such as a portable or handheld medical device, may, however, only have a limited user interface, if any, usually having only a few buttons and a display or just LEDs. Because of such a restricted user interface, there is no capability to easily enter access data as to a wireless connection for the medical device, such as a password or a passcode for a Wi-Fi access point. Therefore, conventional medical devices are restricted to automatic settings of wireless communication channels using wireless protocols without the need of entering access data. As such, conventional medical devices are also restricted to setting up one wireless communication channel and do not have the capability to set up a plurality of wireless communication channels simultaneously and to transfer data (configuration data, sensor data, and the like) via different channels. There is thus a need to improve the setup of wireless connections for a medical device.

### Solution

According to an embodiment, a medical device comprises at least one sensor for generating data related to a usage and/or operation of the medical device and/or data of one or more measured parameters (e.g. measured health parameters, such as therapy-related measurement data) of a user; a connectivity controller configured to: receive access data to a wireless access point from the communication device, and establish a wireless communication connection with the wireless access point using the received access data to wireless transmit the generated data.

According to a preferred embodiment, the connectivity controller is configured to scan an environment of the medical device for a presence of wireless access points.

According to a preferred embodiment, the scanning of the environment of the medical device for the presence of wireless access points is initiated by the connectivity controller in response to an action from the communication device.

According to a preferred embodiment, the connectivity controller is configured to select a subset of (available) wireless access points according to a measure of signal strength.

According to a preferred embodiment, the connectivity controller is configured to select a subset of (available) wireless access points according to a data communication security level.

According to a preferred embodiment, the connectivity controller is configured to filter out wireless access points having a data communication security level lower than the selected subset of wireless access points.

According to a preferred embodiment, the connectivity controller is configured to report the subset of wireless access points to the communication device and receive respective access data for one or more of the subset of wireless access points.

According to a preferred embodiment, the connectivity controller is configured to store the received access data.

According to a preferred embodiment, the established wireless communication connection is used independently of another communication connection with the communication device to transmit the generated data.

According to a preferred embodiment, the establised wireless communication connection is related to one of a Wi-Fi access point, a LoRa access point, a GSM/LTE/6G (radio) access point, a Bluetooth access point, a NFC accees point, an UWB access point or a LPWAN access point.

According to a preferred embodiment, the medical device is an inhalation device configured to generate aerosol and deliver the generated aerosol to a user.

According to a preferred embodiment, the medical device is a lung parameter determination device configured to determine a lung parameter, preferably to measure a volume and/or flow related inhalation or exhalation parameter.

According to a preferred embodiment, a communication device for providing a remote connectivity setup for a medical device comprises: a controller configured to: receive information of one or more wireless access points to be wirelessly connectable with a medical device of one of the above embodiments, select an access point of the one or more wireless access points, and send access data of the selected access point to the medical device.

According to a preferred embodiment, the selection uses a signal strength and/or data security condition.

According to a preferred embodiment, the communication device is a smartphone, a tablet, or a portable computer.

### Brief Description of Drawings

**Figure 1** illustrates a medical device, a communication device and a wireless access point according to a preferred embodiment.
**Figure 2** illustrates a process for establishing a wireless communication connection at the medical device according to a preferred embodiment.
**Figure 3** illustrates a process for establishing a wireless communication connection at the medical device interacting with a communication device according to a preferred embodiment.
**Figure 4** illustrates a process for selecting a wireless access point for a medical device according to a preferred embodiment.

### Detailed Description

Embodiments of the present invention are described with reference to the Figures. It is noted that the following description should not be construed as limiting the invention. In the following and the above, similar or same reference signs indicate similar or same elements or operations.

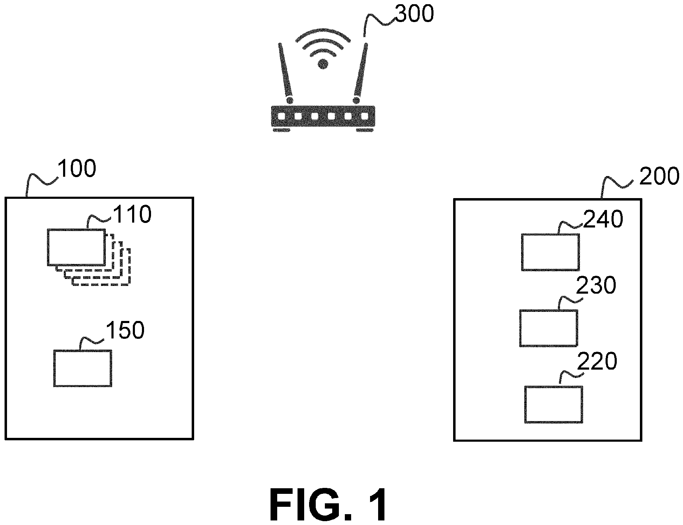

**Figure 1** illustrates a medical device 100, a communication device 200 and a wireless access point 300 according to a preferred embodiment.

The medical device 100 may be any medical device for monitoring or measuring a health state of a patient and/or for providing or delivering a medical dose or drug to a patient, in particular a portable or handheld medical device being wirelessly connectable. That is, the medical device may continuously monitor vital signs of the patient such as heart rate, blood pressure, oxygen saturation and the like. The medical device may also measure or detect parameters related to the lung function (for example, forced expiratory volume, forced vital capacity, vital capacity, tidal volume, peak expiratory flow, respiratory rate). By constantly monitoring such health parameters, early signs of health issues or health trends may be identified, promoting prevention or early intervention which can prevent health complications. Further, such a medical drug delivery device may be configured to administer medications, fluids, aerosols, or other therapeutic agents to a patient in a controlled manner, for example as a precise dosage. The administration may be in the form of a targeted injection into the bloodstream, muscle, or tissue or a delivery into lungs (such as inhalers, nebulizers) or for oral intake. Additionally, the medical device 100 is not provided with a user interface that is sufficient to easily enter access data as to a wireless connection for the medical device, such as a password for the access point 300.

The communication device 200 may be any mobile computing device having telecommunication (wireless data and/or signal transmission) capabilities such as a smartphone, a tablet or the like, in particular having a suitable user interface, for example a graphical user interface, or a voice interface, gesture interface, haptic interface or the like, for entering access data regarding a wireless connection to the wireless access point 300. The medical device 100 and the communication device 200 are distinct different devices. As such, the communication device 200 may be considered as a communication device remote to the medical device.

The wireless access point 300, also referred to as access point (AP), may be a networking hardware device that may allow the medical device 100 and/or the communication device 200 to connect to a wired network or wireless network. As a standalone device, the AP 300 may have a wired or wireless connection to a main network infrastructure, such as a router or switch, but, e.g. in a wireless router, it can also be an integral component of the networking device itself. The wireless access point 300 may employ a Wi-Fi protocol, such as 802.11ax (Wi-Fi 6), but may also provide backward compatibility with older Wi-Fi protocols such as 802.11n (Wi-Fi 4) or 802.11 ac (Wi-Fi 5). As illustrated, also the wireless access point 300 is a hardware entity different from the medical device 100 and the communication device 200. The wireless access point 300 may also be a long range wide area (LoRa) access point, a GSM/LTE/5G radio access point, a near field communication (NFC) access point, an ultra-wideband (UWB) access point (access point using higher frequencies) or a low power wide area network (LPWAN) access point.

The skilled person understands that the medical device 100 may generate a plurality of data sets related to the usage and/or operation of the medical device and/or of one or more measured (health) parameters of the patient. In order to use these data for a continued monitoring, guidance, early detection and prevention of health complications, adherence or the like, the data may need to be transmitted to the communication device 200 which can be configured, e.g. by a suitable programming, a (trained) artificial neural network or the like, to provide such functionalities. The data set may also be transmitted to a data center, e.g. related to a medical institution, with a more powerful computing infrastructure to provide such functionalities. If the wireless communication channel between the medical device 100 and the communication device 200 is not readily available (because the communication device 200 has switched-off the wireless communication channel, is out of reach of the medical device 100, or is powered off), has a limited bandwidth, or the like, it is preferred that the medical device 100 can use an additional wireless communication channel via the wireless access point 300. While, as explained, the medical device 100 itself does not have a suitable user interface to setup a communication connection between the medical device and the wireless access point 300, the present disclosure teaches a remote connectivity setup mechanism to use an external device (here, the communication device 200) with an extended user interface to communicate via a wireless communication connection to the medical device and to thereby transmit the access data, entered at the user interface of the external device or otherwise stored or acquired at the external communication device, to the medical device. By transmitting the access data to the medical device, the medical device may successfully establish a wireless connection with the access point and exchange data via the access point. By this mechanism, the technical limitations of the medical device in terms of user interfaces can suitably be compensated.

In an embodiment of this mechanism, as illustrated in **Figure 1**, the medical device 100 includes at least one sensor 110 and a connectivity controller 150. The communication device 200 includes a wireless interface 220, a controller 230, and a user interface 240.

The at least one sensor 110 may be a sensor acquiring data of a usage and/or operation of the medical device 100 and/or data of one or more measured parameters of the patient. The data of the usage of the medical device 100 may be data indicating how a patient uses or holds the medical device and may include data related to a time of using the medical device, a geographic position of the medical device, an orientation or acceleration of the medical device, a dosage amount delivered by the medical device and the like. The data of the operation of the medical device 100 may be data indicating operation parameters or operation states, such as electrical or optical operation parameters reflecting, for example, a delivery mechanism or delivery conditions for generating a medical dose, or parameters related to power consumption or current output, environmental conditions such as temperature, humidity, pollution of ambient air, air pressure. The data of one or more measured parameters of the patient may be parameters indicating vital parameters of the patient, lung parameters, breathing parameters or the like. The skilled person understands that separate and multiple sensors may be provided to acquire different aspects of using and/or operating the medical device and acquiring patient data.

The connectivity controller 150 of the medical device may control a wireless communication with the communication device 200, in particular together with the wireless interface 220 of the communication device 200. The connectivity controller 150 may be a hardware component (such as a processor, microprocessor, also including associated communication units such as RF transceivers, antennas, filters, and the like) or software module, or a combination thereof, to manage and setup wireless connections, in particular to establish, maintain and switch wireless connections. The connectivity controller 150 may be configured to manage multiple wireless protocols and to handle transitions between different connectivity types to ensure a stable communication. The wireless communication may be based on the Bluetooth standard (IEEE 802.15) the like. In particular, the connectivity controller 150 may perform a wireless communication with the communication device 200 by triggering or initiating the wireless communication, for example using a Bluetooth-based pairing procedure to set up a wireless communication channel. The wireless communication with the communication device 200 may be with regard to accessing the wireless access point 300. The skilled person understands that the wireless communication may also be initiated by the communication device 200, for example when the communication device detects the medical device 100. Setting up the wireless communication between the medical device 100 and the communication device 200 may be performed automatically, in particular without input from a user at the medical device 100 which may only have a limited interface not suitable for manually entering control commands, extended data or the like. In other words, the medical device 100 is not provided with a graphical user interface, a user interface for alphanumeric user input, an interface (camera) for barcode input or the like.

The communication device 200 may use a software application (also referred to as APP) specifically configured for use with the medical device 100. Running or executing the APP at the communication device 200, in particular by the controller 230, may cause an operation interaction between the wireless interface 220, the controller 230 and the user interface 240 of the communication device 200. The controller 230 may be a hardware component, such as a microprocessor, a dedicated chipset or other integrated circuit, that, by using appropriate software and/or firmware, controls specific functions or resources within the communication device 200 by handling individual tasks. The controller 230 may work alongside a central processing unit (CPU). In addition, the user interface 240 may be a graphical user interface, GUI, such as a touch interface, for a user to enter alphanumerical access data and/or other special characters as access data for the wireless access point 300, or a user interface for acquiring the access data via image recognition of a barcode, a fingerprint, face recognition or the like. In other words, the access data to establish a wireless connection between the medical device 100 and the wireless access point 300 can be input via the user interface 240 at the communication device 200.

In a preferred embodiment, the access data may also include an indication of an access point 300. This may be useful in case the communication device 200 has improved capabilities (as compared to the medical device) to scan and detect the environment for available wireless access points.

For the medical device 100 to access the wired/wireless network via the wireless access point 300, the access data are required. Such access data may depend on the wireless interface to the wireless access point 300, and may be Wi-Fi access data, for example an AP name (service set identifier, SSID) and password of the AP, username and password, an access code, access credentials or the like. While the medical device 100 does not provide a sufficient configuration or interface for a user to easily enter such access data, as described above, the connectivity controller 150 may further receive the access data to the wireless access point 300 from the communication device 200, in particular in response to the initiating of a communication between the connectivity controller 150 and the communication device 200. The received access data may be locally stored in the medical device 100, for example in a local storage medium (not shown), and may subsequently be used by the medical device 100.

The connectivity controller 150 may further establish a wireless communication connection with the wireless access point 300 using the received access data. The sensor data generated by the at least one sensor 110 may thus be transmitted to the wireless access point 300 via the established wireless communication connection. Therefore, even if the medical device 100 has not a suitable interface for inputting the access data, the connectivity controller 150 may nevertheless receive the appropriate access data and establish a wireless communication connection between the medical device 100 and the wireless access point 300. As such, a further wireless communication channel (in addition to the one between the medical device 100 and the communication device 200) may be independently established. As the remote connectivity setup mechanism provides suitable access data to the medical device 100 without requiring a corresponding user interface at the medical device 100, the access data may be used subsequently to provide wireless data connection and thus the capability to exchange sensor data with a central computation infrastructure or cloud service without requiring the communication device to be powered on or be present. Alternatively, having two different wireless communication connections at disposal, the set of sensor data may suitably be distributed to the different wireless communication connections, for example based on available bandwidth, data requirements, real-time feedback requirements, and the like. For example, raw data required for a real-time feedback may preferably be transmitted via the established wireless communication connection with the wireless access point 300. In that way, the medical device 100 may directly communicate data required for a real-time feedback to the wireless access point 300 using higher data rates (as compared to using a slower data communication rate between the medical device 100 and the communication device 200.

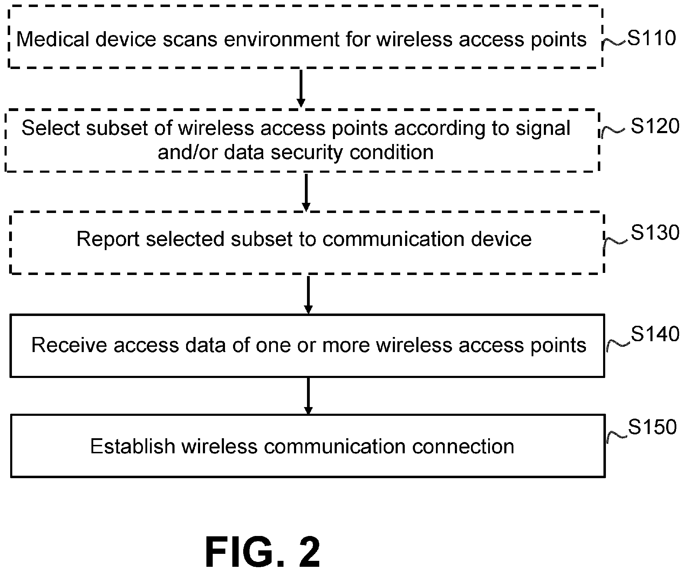

**Figure 2** illustrates a process for establishing a wireless communication connection at the medical device 100 according to a preferred embodiment. According to act S110, the connectivity controller 150 of the medical device 100 may scan an environment of the medical device 100 for a presence of one or more wireless access points. The environment of the medical device 100 may be a local spatial region around the medical device 100, and scanning the environment may identify one or more wireless access points 150. Naturally, the identified wireless access points 150 may be different wireless access points of different architecture or may use different wireless communication and/or data security standards. They may also be positioned at different distances from the medical device and may thus exhibit different signal strengths or the like. The connectivity controller 150 may be configured to determine a wireless access point to be present if the wireless access point provides a signal strength or data rate larger than a set threshold, and/or if the wireless access point uses a specific communication standard, and/or if the wireless access point provides a prescribed data communication security, or the like. For example, a mesh network (a plurality of access points having the same access data), e.g. a Wi-Fi Mesh Network of a plurality of Wi-Fi access points having a same SSID, may be scanned and detected, and the access point having a highest signal strength may automatically be selected.

Preferably, the connectivity controller 150 initiates or starts the scanning of the environment in response to an action received from the communication device 200. In other words, the user interface 240 of the communication device 200, for example when acting on the APP for the medical device 100, may be used to input a command action to trigger the scanning operation at the medical device 100. The command action may be suitably processed at the controller 230 of the communication device 200 and transmitted to the connectivity controller 150 of the medical device 100. In response to receiving the processed command action, the connectivity controller 150 may then proceed to scan the environment to identify whether wireless access points are present.

Referring back to **Figure 2**, the connectivity controller 150 may subsequently, in act S120, select a subset of wireless access points according to a prescribed signal and/or data security condition.

For example, the connectivity controller 150 may select a subset of wireless access points according to a detected signal strength of the corresponding wireless communication connection (network). Selecting only a subset of all available wireless access points, for example only selecting wireless access points having a signal strength larger than a set threshold or a predetermined number of wireless access points having the largest detected signal strengths, may suitably reduce an operation and communication load in order to provide suitable access data. Selected wireless access points may, for example, have a signal strength larger of at least -70 dBm, more preferably at least -60 dBm.

The connectivity controller 150 may also or alternatively select a subset of wireless access points according to a prescribed data communication security level. There may be different data communication security levels, for example, with regard to one or more of encryption, authentication, network segmentation, secure communication protocols (e.g. HTTPS, SSL/TLS). For example, while different Wi-Fi Protected Access levels (WPA) or different Advanced Encryption Standards (AES) may be available, the selected subset of wireless access points preferably should have a prescribed minimum amount of communication security in terms of encryption, e.g. WPA2, in order to protect the generated sensor data from unauthorized access, interception or tampering. In addition, while different authentication mechanism may be available such as 802.1X authentication, multi-factor authentication, pre-shared keys, or the like, the selected subset of wireless access points preferably should have a prescribed minimum amount of communication security in terms of authentication.

Preferably, the connectivity controller 150 may further filter out wireless access points having a data communication security level lower than the selected subset of wireless access points. The connectivity controller 150 may filter out such wireless access points by implementing a multi-step evaluation process. First, the connectivity controller 150 may scan for available wireless networks and acquire details about each detected wireless access point, including the encryption type (e.g., WPA3, WPA2, WEP), the authentication method (e.g., 802.1X, Pre-Shared Key), and any additional security features (e.g., MAC address filtering). The connectivity controller 150 may then compare these details against a predefined security threshold, which specifies acceptable encryption and/or authentication standards. The wireless access points that do not meet or exceed this threshold are automatically excluded from the list of available networks. This process ensures not only that the medical device only connects to networks (access points) that offer a prescribed level of data security, protecting it from vulnerabilities associated with weaker security configurations, but also reduces a communication load between the medical device 100 and the communication device 200 to exchange access data for wireless access points that should not be used.

Referring back to **Figure 2**, the connectivity controller 150 may subsequently, in act S130, perform a communication with the communication device 200 regarding the selected subset of wireless access points. In particular, the connectivity controller 150 may act to report the determined subset of wireless access points to the communication device 200, preferably together with information indicating the detected respective signal strength and/or data communication security level. The controller 230 of the communication device 200 may indicate the determined subset of wireless access points as received from the medical device 100 at the user interface 240, preferably in an ordered list according to the detected respective signal strength and/or data communication security level, and requests the user of the communication device 200 to enter, via the user interface 240, access data for one or more of the subset of wireless access points. The user may readily identify preferred wireless access point(s) based on the ordered list (e.g. wireless access point(s) having the best signal strength(s) and/or highest communication security level(s)), and thus enter access data for the preferred wireless access point(s).

In act S140 of **Figure 2**, the connectivity controller 150 may then receive the access data for one or more of the wireless access points 300 of the subset, for which the access data have been entered at the communication device 200. As described above, the access data may be an access code, name (SSID) and password for the one or more wireless codes, or the like. The received access data may be stored persistently at the medical device, for example, in a flash memory, an EEPROM, a RAM or the like (not shown).

In act S150 of **Figure 2**, the connectivity controller 150 may further use at least one of the received one or more access data (access codes) to establish a wireless communication connection with a corresponding wireless access point 300. In addition, having received access data for more than one wireless access point, the connectivity controller 150 may further switch from a first wireless access point to a second wireless access point, for example when the signal strength of the second wireless access point becomes higher or a data rate for the first wireless access point decreases. Such a scenario may, for example, occur, when a user moves while using the medical device and therefore the spatial relationship between the medical device 100 and wireless access points dynamically changes. The skilled person understands that this supports maintaining a stable wireless connection (in addition to the wireless connection with the communication device), which may be useful for providing continuous real-time feedback.

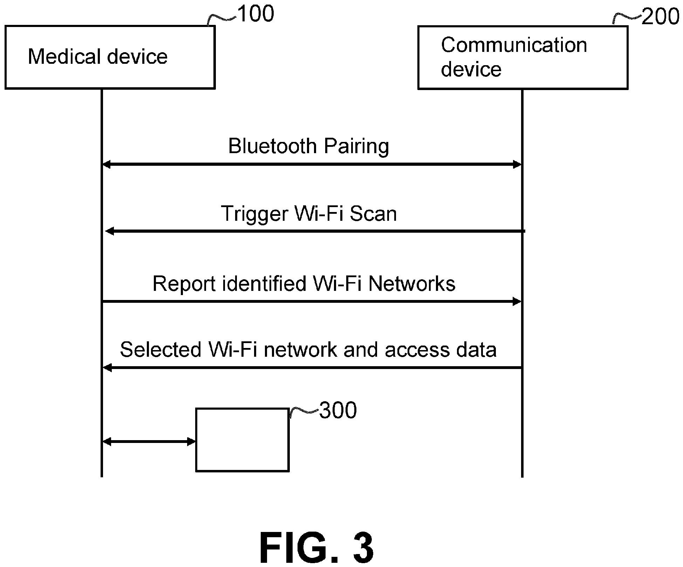

**Figure 3** illustrates a process for establishing a wireless communication connection at the medical device 100 interacting with the communication device 200 according to a preferred embodiment. The process exemplifies the remote connectivity setup mechanism of the present disclosure on the basis of a Bluetooth connection between the medical device 100 and communication device 200 and the wireless access point implementing a Wi-Fi network. The skilled person understands that this is a non-limiting example and that any other wireless communication protocol(s) may equally be applied.

According to **Figure 3**, in a first step, a Bluetooth pairing between the medical device 100 and the communication device 200 may be performed to establish a wireless communication interaction. The Bluetooth pairing may be initiated by either the medical device 100 or the communication device 200, for example in regular time intervals, when one of the devices is powered on, when one of the devices detects the other or the like.

According to a second step of **Figure 3** the communication device 200 may transmit a command to the medical device 100 to trigger Wi-Fi scans, that is, to detect whether wireless access points are present in the environment of the medical device. The command to trigger such Wi-Fi scans may be entered by the user at the user interface 240 and may be communicated via the wireless interface 220 of the communication device 200 to the medical device 100. The connectivity controller 150 may subsequently perform one or multiple Wi-Fi scans, detect one or more wireless access points 300 and may also filter out wireless access points having lower signal strength and/or low data security levels (e.g. lower WPA encryption).

According to a third step of **Figure 3** the connectivity controller 150 may then act to report the identified suitable (having sufficient signal strength and/or required data security level) wireless access points to the communication device 200, preferably together with information of corresponding signal strength and/or data security level. The user interface 240 of the communication device 200 may display the identified Wi-Fi networks (wireless access points) and provide a user interface control for the user to select one (or more) of the Wi-Fi networks and input corresponding access data.

According to a fourth step of **Figure 3** the communication device 200 may then report the selected Wi-Fi network together with the access data to the connectivity controller 150 of the medical device 100. The access data may be stored in the medical device 100, as described above, and the connectivity controller 150 may establish a Wi-Fi connection with the selected wireless access point 300 using the received access data.

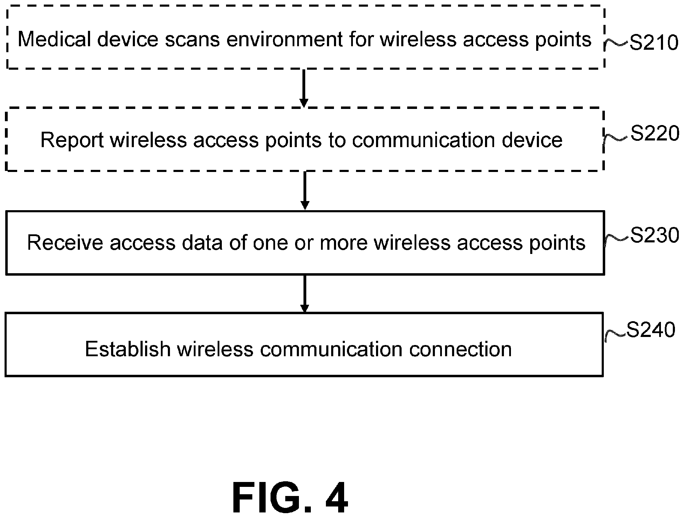

**Figure 4** illustrates a process for selecting a wireless access point for a medical device according to a preferred embodiment.

According to act S210 of **Figure 4** (as in act S110 above), the connectivity controller 150 of the medical device 100 may scan an environment of the medical device 100 for a presence of wireless access points.

Further, according to act S220 of **Figure 4**, the connectivity controller 150 may then report the detected wireless access points to the communication device 200 without selecting a subset of wireless access points, filtering out wireless access points or the like. In particular, this process may be implemented in a case in which the medical device itself is not capable of determining a signal strength and/or data security level. In this case, the selection of the wireless access points according to a signal and/or data security condition, as described above in step S120 of **Figure 2** may be performed by the controller 230 of the communication device. After selecting the subset of wireless access points at the communication device 200, the user interface 240 of the communication device 200 may then display the selected subset of wireless access points, preferably as an ordered list, and a user interface control may be used for the user to select one (or more) of the wireless access points and to input corresponding access data.

Further, according to act S230 of **Figure 4**, the connectivity controller 150 may subsequently receive access data of one or more wireless access points which may be a subset of wireless access points detected and reported in step S220. Receiving access data for such a subset may indicate to the connectivity controller 150 that only information related to this subset may be retained and to discard connection information of wireless access points outside of the subset.

Further, according to act S240 of **Figure 4**, the connectivity controller 150 may then use at least one of the received one or more wireless data to establish a wireless communication connection with a corresponding wireless access point. In addition, as described above, having received access data for more than one wireless access point, the connectivity controller 150 may further switch from a first wireless access point to a second wireless access point, for example when the signal strength of the second wireless access point becomes higher. The switch from a first wireless access point to a second wireless access point may also be triggered or instructed by the communication device 200, for example by a corresponding user interaction at the user interface 240 or by a control command issued by the controller 230. For example, the controller 230 may also scan the environment and evaluate available access points as to signal strength and/or data communication security level, and determine an optimal access point.

In a further embodiment, the medical device may be an inhalation device configured to generate aerosol and deliver the generated aerosol to a user. In another embodiment, the medical device may be a lung parameter determination device. The lung parameter determination device may, for example, determine a breathing frequency or oxygen saturation. The lung parameter determination device may, for example, be a spirometer configured to measure a volume and/or flow related inhalation or exhalation parameter, such as vital capacity, forced respiratory volume in one second and the like. The skilled person knows that there are various types of spirometer using a number of different sensor unists for measurement, for example, a pressure tzransducer, an ultrasonic transmitter, a hot-wire anemometer, a turbine (opical, acoustic) generating therapy-related data and the like. The lung parameter determination device may also be a flow meter to determine a (peak) expiratory flow, or a pulse oximeter to determine blood oxygen saturation indirectly assessing lung function. The lung parameter determination device may also be a capnometer to determine a concentration of carbon dioxide in exhaled air. The remote connectivity setup mechanism for the inhalation device or the lung parameter determination device allows to establish a (separate) secure and stable wireless communication connection which otherwise would require complex user inputs at the medical device to enter access details. Sensor data acquired by sensors in the inhalation device or the lung parameter determination device may thus be transmitted, completely and promtply, and thus may be used for providing improved feed-back, continous user guidance or the like.

The above respective units of the medical device 100 and/or the communication device 200 may be implemented by a respective processing unit (CPU) that includes one or a plurality of processors, a microprocessor or other processing logic that interprets and executes instructions as defined by the computer program and stored in a main memory. The main memory may include a RAM or other type of dynamic storage device that may store information and instructions for execution by the respective modules/units. For example, the evaluation unit and/or the sensor data control unit discussed above may be realized by the processing unit. The ROM may include a ROM device or another type of static storage device that may store static information and instructions for use by the processing unit.

The medical device 100 and/or the communication device 200 may perform these operations in response to the processing unit executing software instructions contained in a computer-readable medium, such as the main memory, ROM and/or storage device. A computer-readable medium may be defined as a physical or a logical memory device. For example, a logical memory device may include memories within a single physical memory device or distributed across multiple physical memory devices. Each of the main memory, ROM and storage device may include computer-readable media with instructions as program code. The software instructions may be read into the main memory for another computer-readable medium, such as a storage device or from another device via the communication interface.

The software instructions contained in the main memory may cause the processing unit(s) including a data processor, when executed on the processing unit, to cause the data processor to perform operations or processes described herein. Alternatively, hard-wired circuitry may be used in place or in combination with the software instructions to implement processes and/or operations described herein. Thus, implementations described herein are not limited to any specific combination of hardware and software.

Further, the respective units of the medical device 100 and/or the communication device 200 may be implemented in hardware, software, Field Programmable Gate Arrays (FPGAs), application-specific integrated circuits (ASICs), firmware or the like.

In the foregoing detailed description of the present disclosure, reference is made to the accompanying drawings that form a part hereof, and in which is shown by way of illustration how examples of the disclosure may be practiced. These examples are described in sufficient detail to enable those of ordinary skill in the art to practice the examples of this disclosure, and it is to be understood that other examples may be utilized and that process, electrical, and/or structural changes may be made without departing from the scope of the present disclosure. Elements shown in the various figures herein can be added, exchanged, and/or eliminated to provide a number of additional examples of the present disclosure. In addition, the proportion and the relative scale of the elements provided in the figures are intended to illustrate the examples of the present disclosure and should not be taken in a limiting sense.

## Claims

1. A medical device (100), comprising
at least one sensor (110) for generating data related to a usage and/or operation of the medical device (100) and/or data of one or more measured parameters of a user; and
a connectivity controller (150) configured to:
- receive access data to a wireless access point (300) from a communication device (200), and
- establish a wireless communication connection with the wireless access point (300) using the received access data to wireless transmit the generated data.

2. The medical device (100) of claim 1, wherein the connectivity controller (150) is further configured to scan an environment of the medical device (100) for a presence of wireless access points.

3. The medical device (100) of claim 2, wherein the scanning of the environment of the medical device (100) for the presence of wireless access points is initiated by the connectivity controller (150) in response to an action from the communication device (200).

4. The medical device (100) of one of claims 1 - 3, wherein the connectivity controller (150) is further configured to select a subset of wireless access points according to a measure of signal strength.

5. The medical device (100) of one of claims 1 - 4, wherein the connectivity controller (150) is further configured to select a subset of wireless access points according to a data communication security level.

6. The medical device (100) of claim 5, wherein the connectivity controller (150) is further configured to filter out wireless access points having a data communication security level lower than the selected subset of wireless access points.

7. The medical device (100) of one of claims 4 - 6, wherein the connectivity controller (150) is further configured to report the subset of wireless access points to the ommunication device (200) and receive respective access data for one or more of the subset of wireless access points.

8. The medical device (100) of claim 7, wherein the connectivity controller (150) is further configured to store the received access data.

9. The medical device (100) of one of claims 1 - 8, wherein the established wireless communication connection is used independently of another communication connection with the communication device to transmit the generated data.

10. The medical device (100) of one of claims 1 - 9, wherein the establised wireless communication connection is one of related to a Wi-Fi access point, a LoRa access point, a GSM/LTE access point, a Bluetooth access point, a NFC accees point, a UWB access point or a LPWAN access point.

11. The medical device (100) of one of claims 1 - 10, wherein the medical device is an inhalation device configured to generate aerosol and deliver the generated aerosol to a user.

12. The medical device (100) of one of claims 1 - 10, wherein the medical device is a lung parameter determination device configured to measure a volume and/or flow related inhalation or exhalation parameter.

13. A communication device (200) for providing a remote connectivity setup for a medical device (100), comprising
a controller (230) configured to:
- receive information of one or more wireless access points to be wirelessly connectable with a medical device of one of claims 1 - 12,
- select an access point of the one or more wireless access points, and
- send access data of the selected access point to the medical device (100).

14. The communication device (200) of claim 13, wherein the selection uses a signal strength and/or data security condition.

15. The communication device (200) of one of claims 13 - 14, wherein the communication device is a smartphone, a tablet, or a portable computer.

16. A computer program, comprising instructions which, when executed on at least one processor of a computer, cause the at least one processor to function as a medical device according to any of claims 1 - 12 or a communication device according to any of claims 13 - 15.

17. A computer-readable storage medium, having stored thereon a computer program according to claim 16.

18. A carrier containing the computer program according to claim 17, wherein the carrier is one of an electrical signal, optical signal, radio signal, or computer-readable storage medium.
